# EUROPEAN PATENT SPECIFICATION

(11) **EP 0 371 574 B1**
(45) Date of publication and mention of the grant of the patent: **17.05.1995**
(21) Application number: 89302373.9
(22) Date of filing: 10.03.1989
(51) Int. Cl.: A61B 10/00, A61F 13/20, C12M 1/30

(54) **Swab transport apparatus**
Tupfer-Transportvorrichtung
Dispositif pour le transport de tampons

(30) Priority: 28.11.1988 US 276639; 23.12.1988 US 288987
(43) Date of publication of application: 06.06.1990
(73) Proprietor: ST. AMAND MANUFACTURING, INC., San Fernando California (US)
(72) Inventor: Saint-Amand, Elmer Felix, Canyon Country California 91351 (US)
(74) Representative: Boon, Graham Anthony

(56) References cited:
- EP-A- 0 155 747
- US-A- 3 163 160
- US-A- 3 918 435

## Description

The present invention relates generally to a disposable swab transport apparatus. More particularly, the invention concerns an apparatus for obtaining a sample or organisms, such as bacteria, which may be present in or on various parts of the body and preserving the same by immersing it within a selected transport medium during transport of the sample to a clinical laboratory for testing. The transport medium can be added to the apparatus at time of manufacture or, alternatively, can conveniently be added to the apparatus shortly prior to use.

It is frequently necessary for a physician to obtain a sample of an organism from a patient. As a general rule, the physician removes a sterile swab from its supply package, contacts the area from which the specimen is to be taken and then forwards the swab to a laboratory for testing. Without the exercise of great care during transport, the specimen can become contaminated with foreign particles or organisms and the swab, itself can undesirably spread contamination on route to the laboratory. Further, unless the specimen is maintained in a viable state, such as by treatment of the swab with an appropriate preservative medium, an accurate analysis cannot be obtained.

In the past efforts have been made to provide a disposable swab container within which the transport medium is prepackaged. One such prior art apparatus consists of a two section tube having a swab in one section and a supply of transport medium i the other section. A slotted valve is provided between the two sections to permit the swab to be exposed to the transport medium. Another device consists of a flexible tube having a frangible ampoule in one end, absorbent material adjacent the ampoule and a swab disposed in contact with the absorbent material. In using this device a sample is taken with the swab, the swab is inserted into the flexible walled container and the area containing the ampoule is squeezed to break the ampoule. The medium contacts the absorbent material which then moistens the tip of the swab in contact with the absorbent material.

Still another prior art device provides a flexible walled swab enclosure and a closure cap carrying a hollow stemmed swab. A frangible, medium containing ampoule is mounted in the housing. With this device, after the sample is taken, the physician squeezes the housing to fracture the ampoule. The initial squeezing of the housing followed by repeated squeezing of the housing pumps the medium in the swab tip. This latter described device is more fully discussed in U.S. Patent No. 3,918,435, issued to Beall et al. Other prior art apparatus for transporting swabs and specimens is described in U.S. Patent Nos. 3,163,160, 3,282,114, 3,616,265 and 3,674,004.

In most, if not all, of the prior art devices the transport medium is prepackaged within the apparatus, as sold. Accordingly, if different types of transport mediums are required for preserving the different specimens which are to be taken, different apparatus must be purchased and maintained in inventory. Another drawback of the prior art swab transport devices which carry the transport medium during transport and storage is the potential for leakage of the medium from the device and the potential for accidental exposure of the sterile swab to the transport medium prior to the specimen being taken. In either case, the often costly apparatus must be discarded.

EP-A-0155747 describes an apparatus intended to prevent accidental premature exposure of the swab to the transport medium. The apparatus comprises a swab transport apparatus for containing and transporting a swab of the character having an elongated stem and a bibulous swab material carried proximate one end of the stem, said apparatus comprising an elongated plastic body having a longitudinally extending axis, resiliently deformable side walls and first and second ends, said body being open at said first end and including:
(a) a first elongated chamber located proximate said first open end for receiving at least a portion of the swab;
(b) a second chamber having resiliently, deformable side walls for containing fluid therewithin, said second chamber being longitudinally displaced from said first chamber; and
(c) a tubular segment disposed proximate said second end of said body, said segment having a fluid passageway extending therethrough for interconnecting said second chamber with atmosphere.

The apparatus of EP-A-0155747 comprises, for the purpose of preventing the aforesaid premature exposure, a rupturable membrane which is intended to be pierced by the swab.

In contrast, the apparatus of the present invention is characterized in that a reduced diameter portion is disposed intermediate said first and second chambers, said reduced diameter portion being in communication with both said first and second chambers, wherein said reduced diameter portion includes a third chamber having resiliently deformable side walls disposed intermediate said first and second chambers, said third chamber being in communication with said first chamber via an axially extending fluid passageway and said third chamber being in communication with said first chamber via a circuitous fluid flow path.

In particular embodiments thereof, the invention provides a swab transport apparatus as described in the preceding paragraph in which a specimen of the transport fluid which has been exposed to the contaminated swab can be obtained and separately transported to the laboratory for testing.

The swab transport apparatus of the apparatus may comprise a blow molded container having a swab chamber and a transport medium chamber, and a closure cap, all of which can be inexpensively manufactured and then assembled together into a highly compact subassembly which can be easily packaged, used, transported and stored.

The apparatus of the invention may comprise a blow molded container provided with one or more longitudinally extended flat surfaces which provide a writing surface for inscribing appropriate identification data directly on the device at the time a specimen is taken.

Figure 1 is a front view of a swab transport apparatus which, although not according to the claimed invention, is illustrated, and described below, for the purpose of indicating various features which are used in the claimed invention.

Figure 2 is a side elevational view of the apparatus.

Figure 3 is a cross-sectional view taken along lines 3-3 of Figure 1.

Figure 4 is a cross-sectional view taken along lines 4-4 of Figure 2.

Figure 5 is a cross-sectional view taken along lines 5-5 of Figure 4.

Figure 6 is a cross-sectional view taken along lines 6-6 of Figure 4.

Figure 7 is a cross-sectional view taken along lines 7-7 of Figure 4.

Figure 7a is a fragmentary view of one end of the apparatus shown in Figure 3 illustrating the appearance of the device following sealing of the inlet fill tube.

Figure 8 is a front view of a first embodiment of the swab transport apparatus of the present invention.

Figure 9 is a side elevational view of the apparatus of Figure 8.

Figure 10 is a cross-sectional view taken along lines 10-10 of Figure 9.

Figure 11 is a cross-sectional view taken along lines 11-11 of Figure 10.

Figure 12 is a cross-sectional view taken along lines 12-12 of Figure 10.

Figure 13 is a front view of still another form of swab transport apparatus of the present invention.

Figure 14 is a side elevational view of the apparatus shown in Figure 13.

Figure 15 is a cross-sectional view taken along lines 15-15 of Figure 14.

Figure 16 is a cross-sectional view taken along lines 16-16 of Figure 15.

Figure 17 is a cross-sectional view taken along lines 17-17 of Figure 15.

Figure 18 is a cross-sectional view taken along lines 18-18 of Figure 15.

Figure 19 is a front view of yet another embodiment of the apparatus of the present invention.

Figure 20 is a side elevational of the apparatus shown in Figure 19.

Figure 21 is a cross-sectional view taken along lines 21-21 of Figure 20.

Figure 22 is a cross-sectional view taken along lines 22-22 of Figure 21.

Figure 23 is a cross-sectional view taken along lines 23-23 of Figure 21.

Figure 24 is a cross-sectional view taken along lines 24-24 of Figure 21.

Figure 25 is a front view of still another embodiment of the apparatus of the present invention.

Figure 26 is a side elevational view of the apparatus shown in Figure 25.

Figure 27 is a cross-sectional view taken along lines 27-27 of Figure 26.

Figure 28 is a cross-sectional view taken along lines 28028 of Figure 27.

Figure 29 is a cross-sectional view taken along lines 29-29 of Figure 27.

Figure 30 is a cross-sectional view taken along lines 30-30 of Figure 27.

Figure 31 is a front view of yet another embodiment of the apparatus of the present invention.

Figure 32 is a side elevational of the apparatus shown in Figure 31.

Figure 33 is a cross-sectional view taken along lines 33-33 of Figure 32.

Figure 34 is a cross-sectional view taken along lines 34-34 of Figure 33

Figure 35 is a cross-sectional taken along lines 35-35 of Figure 33.

Figure 36 is a cross-sectional view of another embodiment of the apparatus of the invention wherein the lower fluid chamber is axially off-set from the chamber which receives the swab.

Figure 37 is a cross-sectional view taken along lines 37-37 of Figure 36.

Figure 38 is a fragmentary, cross-sectional view of an apparatus similar to that shown in Figure 36, but embodying a novel fluid passageway closing mechanism.

Figure 39 is a fragmentary, cross-sectional view similar to Figure 38, but showing the fluid passageway closing mechanism in a closed configuration.

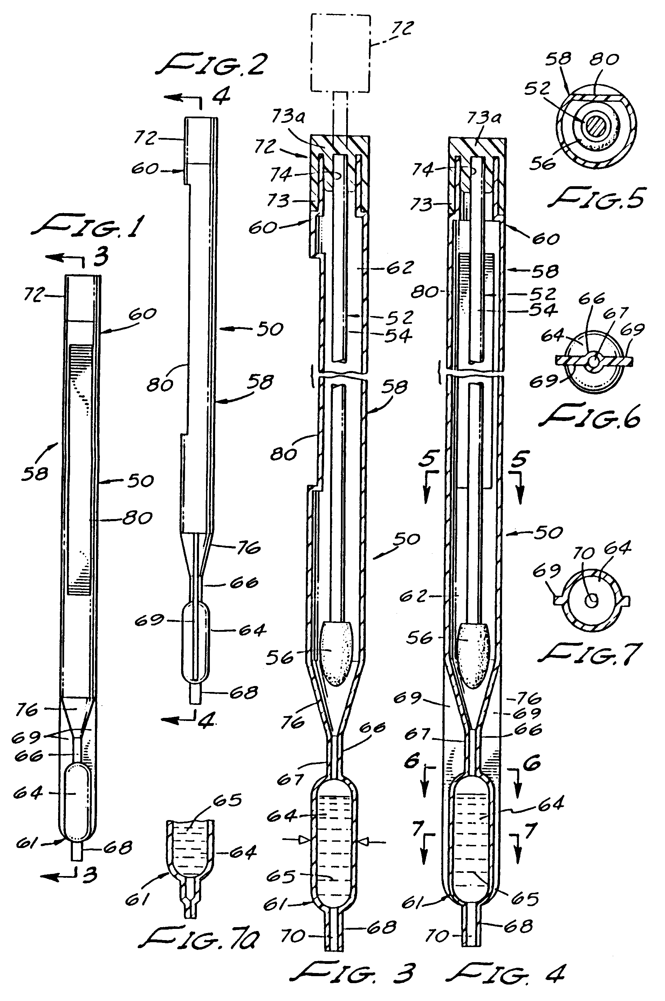

Referring to Figures 1 through 7, the swab transport apparatus thereshown is generally designated by the numeral 50. The apparatus is used for transporting a swab 52 of the character having an elongated stem 54 and a bibulous swab material 56 carried proximate one end of the stem. As best seen by referring to Figures 3 and 4, the apparatus comprises an elongated plastic body 58 having resiliently deformable side walls and first and second end portions 60 and 61. Body 58 is open proximate its first or upper end 60 and includes a first elongated chamber 62 communicating with the open end for receiving the swab 52.

Body 58 also includes a second chamber 64 having resiliently deformable side walls for containing a fluid 65 therewithin. Second chamber 64 functions as a squeeze bulb, is axially aligned with chamber 62 and is longitudinally spaced therefrom. Disposed intermediate chambers 62 and 64 is a reduced diameter portion 66 which includes a communication path between first and second chambers 62 and 64 provided here as an axially extending passageway 67. Disposed proximate second end 61 of the plastic body is a tubular shaped segment 68 having a fluid passageway 70 therethrough for interconnecting second chamber 64 with atmosphere.

A removable first cover means is provided for sealably closing the open first end of body 58. This cover means is here provided as a plastic, cup-shaped member 72 having downwardly depending flexible, skirt like, side walls 73 adapted to sealably engage the side walls of tubular body 58 proximate the open upper end 60 thereof. Cap 72 includes a central portion 73a which is provided with an axially extending central bore 74 adapted to closely receive the upper end of elongated stem 54 of swab 52. With this construction, the swab can be removed from the apparatus by gripping the cap member 72 and simultaneously withdrawing the cap and the swab from the body portion 58 in the manner indicated by the phantom lines in Figure 3. In this way, the sample can be taken without the physician touching any part of the swab itself. After the specimen has been taken, the swab can be reinserted into body 58, once again by handling only cap 72. A downward pressure exerted on cap 72 will cause the skirt, or wall portions, 73 to be received over and closely seal about the walls proximate the open first end 60 of the plastic body 58.

In Figures 3 and 4 it can be seen that body 58 further includes a generally frustoconical shape transition portion 76 disposed intermediate first chamber 62 and reduced diameter portion 66. The lower end of swab portion 56 is partially receivable within this transition portion when cap 72 is fully seated.

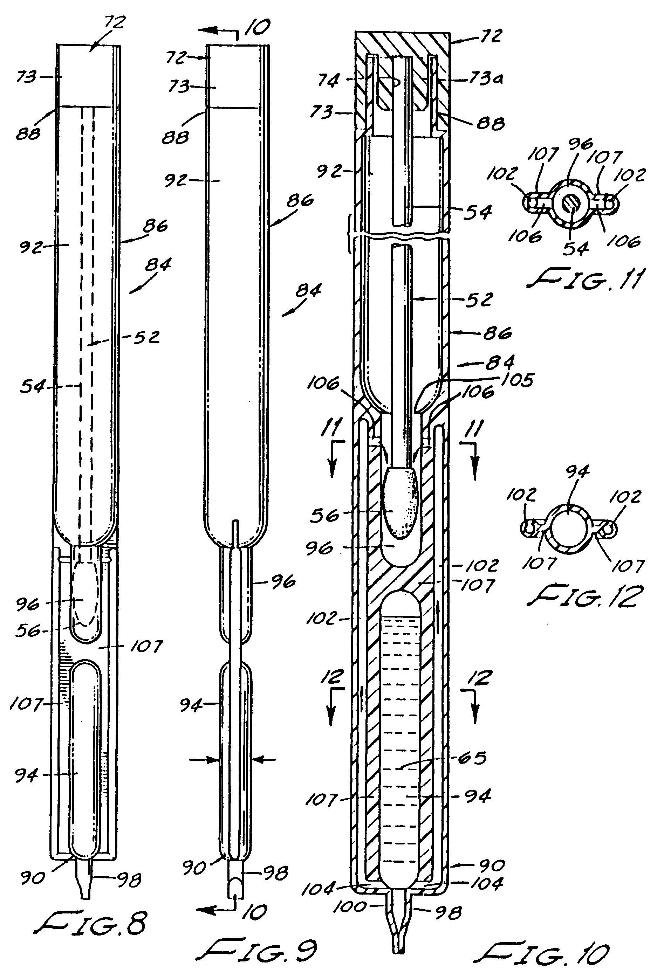

In using the apparatus illustrated in Figures 1 through 7, a fluid, such as a transport medium 65 can be added to chamber 64 at the time of manufacture via the open end of body 58 or through use of a syringe assembly with a needle adapted to penetrate the side walls of chamber 64. Alternatively, the transport medium 65 can be drawn into chamber 64 at any time prior to use by inserting tubular segment 68 into a vial containing the fluid, squeezing the flexible walls of chamber 64 and then releasing them to cause the fluid 65 to be drawn into chamber 64 in the manner shown in Figures 3 and 4. Once the transport medium most appropriate for use in connection with preserving the culture to be taken using the swab 52 is added to chamber 64, tubular segment 68 can be crimped and heat sealed in the manner shown in Figure 7a. This heat sealing step can be accomplished in various ways understood by those skilled in the art and effectively closes off passageway 70 thereby preventing fluid flow through the passageway to the exterior of the apparatus.

After the specimen has been taken by the physician and the swab re-inserted into the apparatus in the manner shown in Figures 3 and 4 and with the tube segment 68 sealed in the manner shown in Figure 7a, squeezing the yieldable side walls of chamber 64 will cause the transport fluid to be injected into the bottom portion of first chamber 62 via passageway 67. Chamber 64 is of a volume such that when the fluid is transferred from chamber 64 into chamber 62, the fluid will substantially encapsulate the bibulous swab material 56 carried by the swab. With the fluid transport medium saturating the bibulous swab material, the entire apparatus can be safely transported to the clinical laboratory for testing.

The ability to add the transfer medium to chamber 64 of the apparatus of the invention as depicted in Figures 1 through 7 via tubular segment 68 is particularly important when the selected transfer medium comprises a viscous liquid such as a gel. When such a medium is used it is virtually impossible to add the medium to chamber 64 via the open upper end 58.

Another useful feature of the apparatus shown in Figures 1 through 7 comprises the provision of a flat portion, or surface, 80 on the body portion 58. As best seen by referring to Figures 1, 2 and 5, portion 80 provides a convenient, smooth writing surface for inscribing thereupon appropriate identification data regarding the specimen taken and carried by the swab 52.

As indicated in the Figures 5, 6 and 7, plastic body portion 58 of the apparatus is substantially circular in cross-section at any point. As best seen in Figure 6, reduced diameter portion 66 of the apparatus is reinforced through the provision of transversely spaced, longitudinally extending web portions 69.

Turning now to Figures 8 through 12, a transport apparatus of the present invention is there shown and generally designated by the numeral 84. The apparatus of this form of the invention is used for transporting an identically configured swab 52 of the character previously described having an elongated stem 54 and a bibulous swab material 56 carried proximate one end of the stem. As best seen by referring to Figure 10, the apparatus comprises an elongated plastic body 86 having yieldably deformable sidewalls and first and second end portions 88 an d 90. Body 86 is open proximate its first or upper end 88 and includes the first elongated longitudinally extending chamber 92, communicating with the open end for receiving the swab 52.

Body 86 also includes a second chamber 94 having yieldable sidewalls for containing a fluid 65 therewithin. As best seen by referring to Figure 9, second chamber 94 functions as a squeeze bulb, is axially aligned with chamber 92 and is longitudinally spaced therefrom. Deposed intermediate chambers 92 and 94, is a reduced diameter portion shown here as an intermediate chamber 96. Chamber 96 has flexible side walls and is in communication with chambers 92 and 94 via uniquely arranged fluid passageways presently to be described.

Disposed proximate second end 90 of the plastic body is a tubular shaped segment 98 having a passage way 100 therethrough for interconnecting second chamber 94 with atmosphere. In the drawings illustrating this second embodiment of the invention, tubular segment 98 is shown in a crimped or sealed configuration. In using the apparatus, chamber 94 is filled with an appropriate transfer medium prior to the sealing step by placing tubular segment 98 into a reservoir of the selected transport medium and then squeezing squeeze bulb or chamber 94 to draw the transport fluid into chamber 94. Once the transport fluid has been drawn into the apparatus, tubular segment 98 is sealed in the manner shown in the drawings. It is to be understood that, instead of sealing the tubular segment 98, as by heat sealing, segment 98 can be sealed using any type of suitable mechanical clamping device.

A removable first cover means having a configuration identical to that previously described is also provided for use in connection with the apparatus of the present form of the invention. This cover means, like that of the previously described embodiment of the invention, comprises a plastic cup-shaped member 72 having downwardly depending flexible sidewalls 73 adapted to sealably engage the sidewalls of tubular body 86 proximate the open upper end 88 thereof. Cap 72 includes the central portion 73 which is provided with an axially extending central bore 74 adapted to closely receive the upper end of the elongated stem 54 of the swab 52. With the construction thus described, the swab can be removed, the specimen taken and the swab reinserted in the manner described in connection with the previous embodiment of the invention.

An important feature of the apparatus of the invention shown in Figure 10 resides in the provision of a circuitous fluid flow path interconnecting intermediate chamber 96 with chamber 94. In the present embodiment of the invention this circuitous fluid flow path comprises transversely spaced, longitudinally extending fluid passageways 102 disposed on opposite sides of chambers 96 and 94 (Figure 10). Proximate the lower extremities of passageways 102 are perpendicularly extending legs or passageways 104 which communicate with the lower extremity of chamber 94. Provided proximate the upper extremities of passageways 102, are vertically extending legs or short passageways 106 which communicate with chamber 96 proximate the upper end thereof. As indicated in Figure 10, chamber 96 communicates with chamber 92 via a centrally disposed axially extending opening 105. With this arrangement, when swab assembly 52 is in position within the apparatus, bibulous portion 56 of the swab is received within chamber 96 and is positioned at a location below the transversely extending passageways 106.

In using the apparatus illustrated in Figure 8 through 12 a fluid such as transport medium 65, if not added at time of manufacture, is drawn into chamber 94 in the manner previous described. Once the transport medium most appropriate for use in connection with preserving the culture to be taken is in place within chamber 94, tubular segment 98 is crimped and heat-sealed as depicted in the drawings. After the specimen has been taken by the physician and the swab is reinserted into the apparatus, squeezing the yieldable sidewalls of chamber 94 will cause the fluid within chamber 94 to be forced through fluid passageways 104 and 102 in the direction shown by the arrows in Figure 10. The fluid will enter chamber 96 via transversely extending legs portions 106 and will saturate the bibulous portion 56 of the swab. It is to be understood that chamber 94 is of a volume sufficiently large so that the transport medium contained therein can be injected into chamber 96 to substantially fill the chamber so as to completely saturate the bibulous portion 56 of the swab during the time the apparatus is being transported to the laboratory for testing.

As indicated in Figures 11 and 12, chambers 94 and 96 are substantially circular in cross section with longitudinally extending passageways 102 being spaced apart from the axially centerline of the chamber and being carried within transversely extending weblike plastic portions 107 (Figures 8 and 10).

As was the case with the earlier described embodiment of the invention, body portion 86 along with tubular segment 98 is integrally formed by means of a blow molding process using, for example, a suitable blow-moldable plastic material selected from the olefin group.

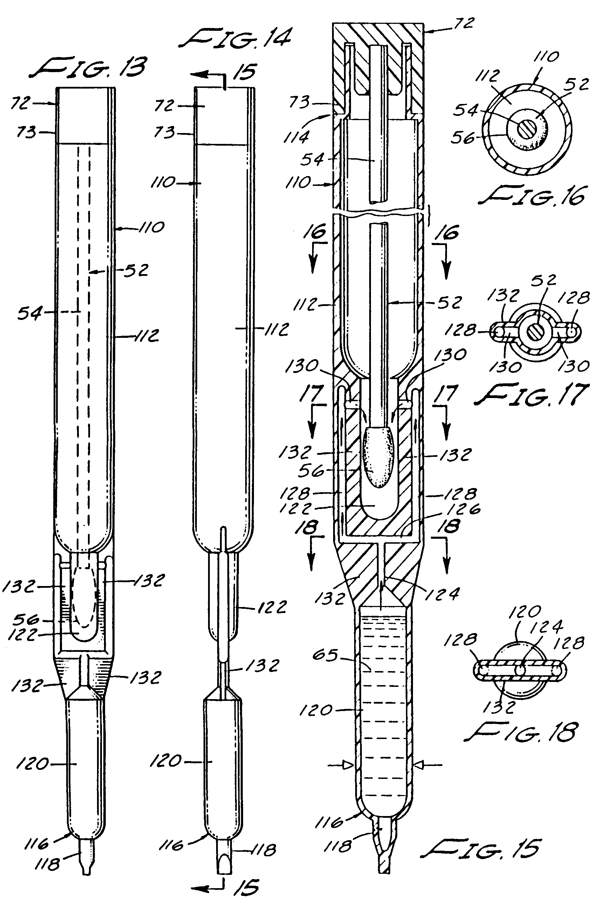

Turning now to Figures 13 through 18, still another embodiment of the apparatus of the invention is there shown. Many portions of the apparatus of this form of the invention are identical to those previously described and the same reference numbers are used to identify components of like construction. For example, as indicated in Figure 15, the cap portion 72 and the swab assembly 52 are of identical construction to that previously described herein save for the fact that swab 52 is provided with a slightly shorter stem and cap 72 is of a slightly larger diameter so as to sealably engage with the sidewalls of the body portion 110 which also is of a slightly larger diameter than the body portion of the transfer apparatus previously described.

The apparatus of this further form of the invention includes a longitudinally extending chamber 112 which is in communication with the upper open end portion 114 of body portion 110. Formed proximate the lower portion 116 of the body portion, is a tubular shaped segment 118 which is used to draw an appropriate transport fluid into a second chamber 120 of this embodiment of the invention. The tubular segment is then sealed in the manner shown in the drawing. The sidewalls of chambers 112 and 120 are yieldably deformable and function as squeeze bulbs.

Disposed intermediate chambers 112 and 120 is a reduced diameter portion including a third chamber comprising squeezably deformable bulb 122. As best seen in Figure 15, in this form of the invention, chambers 112, 120 and 122 are all axially aligned and are in communication with one another via uniquely arranged fluid passageways of a character presently to be described.

Once again, an important aspect of the apparatus of this later form of the invention resides in the provision of a circuitous fluid flow path between chambers 120 and 122. This circuitous flow path prevents the inadvertent premature transfer of the fluid medium 65 from chamber 120 into the swab enclosing intermediate chamber 122 as might occur during handling or vibration of the apparatus.

As indicated in Figure 15, the fluid flow path of the device this embodiment of the invention comprises an axially aligned fluid passageway 124 one end of which communicates with chamber 120 and the other end of which intersects a transversely extending passageway 126. At either end of passageway 126 there is provided longitudinally extending passageways 128 which are located on opposite sides of squeeze bulb 122. Longitudinally extending passageways 128 communicate proximate their upper ends with transversely extending passageway segments 130 which, in turn, communicate with chamber or squeeze bulb 122 proximate the upper end thereof.

In using this latest described form of the apparatus, a fluid such as transport medium 65 is prepackaged with the device, or is drawn into chamber 120 in the manner previously described and tubular segment 118 sealed as shown in the drawings. After the specimen has been taken by the physician and the swab reinserted into the apparatus in the manner shown in Figure 15, squeezing the yieldable sidewalls of squeeze bulb or chamber 120 will cause the transport fluid to be injected into chamber 122 via passageways 124, 126, 128 and 130. Chamber 120 is of such a volume that when the fluid is transferred from chamber 120 into chamber 122, the fluid will substantially encapsulate bibulous swab material 56 which is disposed within chamber 122. With the fluid transport medium having saturated the bibulous swab material, the entire apparatus can be safely transported to the clinical laboratory for testing.

As indicated in Figures 16, 17 and 18, once again the plastic body portion 110 of the apparatus is shown in these drawings is substantially circular in cross section at any point. It is to be noted that fluid passageways 128 are disposed on opposite sides cf chamber 122 and are carried by means of a plastic, flange-like reinforcement 132 (Figures 13, 15, 17 and 18).

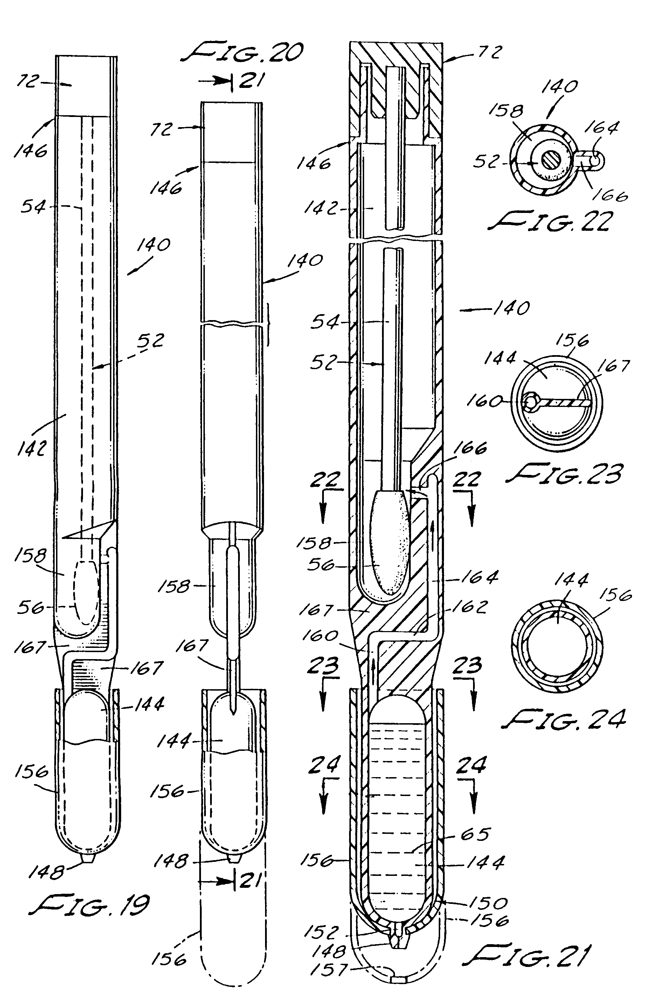

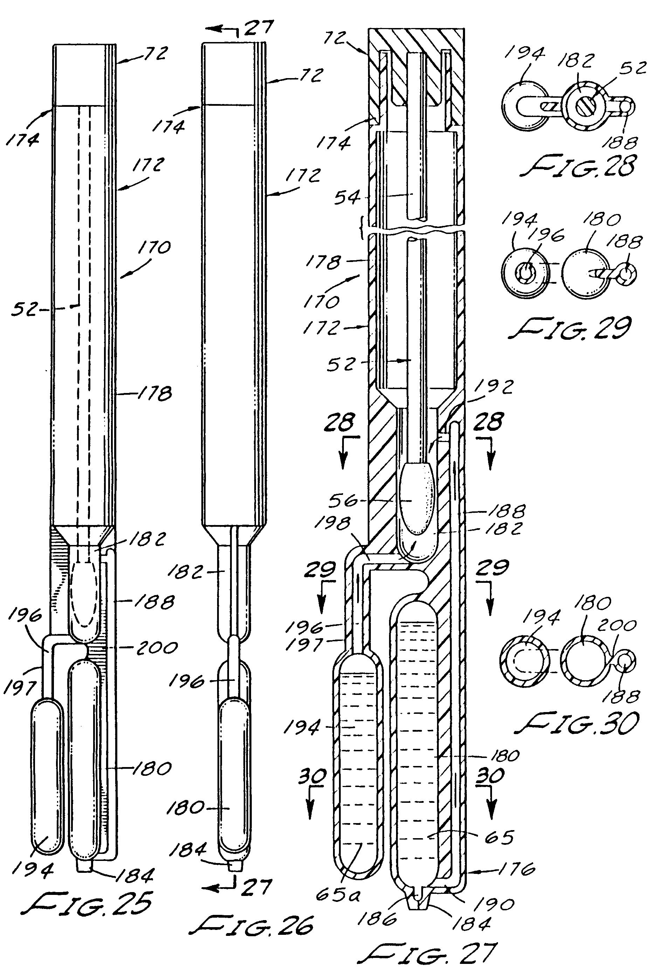

Turning now to Figures 19 through 24, still another form of the apparatus of the invention is there shown. Once again, this form of the apparatus is similar in many respects to the apparatus described in the preceding paragraphs and like numbers are used in the drawings to identify like components. The apparatus here shown comprises an elongated plastic body 140 including longitudinally spaced apart first and second chambers 142 and 144. Plastic body 140 is open proximate its upper end 146 and is provided with a tubular shaped segment 148 proximate its lower extremity 150. Tubular extremity 148 has a fluid passageway 152 which enables communication between 144 and atmosphere.

In the instant form of the invention, tubular section 148, the passageway of which can be sealed by crimping or heat sealing, functions to hold a second cap, or safety shield, 156 in position about the lower squeeze bulb or chamber 144. With this unique construction, when the closure cap 156 is moved from the position shown in the phantom lines in Figure 21 into the position shown in the solid lines, tubular section 148 is closely receivable within a centrally disposed aperture 157 formed in safety shield member 156. As the member 156 is snapped into position about the lower end of the apparatus, its upstanding walls surround the squeeze bulb or chamber 144 thereby serving to positively preclude any inadvertent squeezing of the chamber and the resulting accidental injection of the transport medium into chamber 158.

In using this latter form of the apparatus safety cap 156 is removed, and the appropriate transport fluid 65 drawn into chamber 144 through open passageway 152. After the chamber has been filled and passageway 152 appropriately sealed, safety member or cap 156 is snapped in place thereby surrounding chamber 144 in the manner illustrated in the solid lines of Figure 21.

As was the case with the earlier described embodiments of the invention, this form of the invention also includes an intermediate reduced-diameter portion shown here in the form of a third squeeze bulb or chamber 158. As best seen in Figure 21, chamber 158 is axially off-set and is in communication with first and second chambers 142 and 144 by means of a novel circuitous fluid flow path of a character presently to be described.

Contained within the apparatus of the invention is a removable swab assembly 52 of a configuration virtually identical to that previously described. The upper extremity of swab assembly 52 is receivable within a bore formed in a cap 72 which cap is also of virtually identical construction to the cap used in connection with the previously described embodiments of the invention.

After the specimen has been taken by the physician and the swab reinserted into the apparatus in the manner shown in Figures 19 and 21 and with the cap member 156 closing off passageway 152, squeezing the deformable side walls of chamber 144 will cause the transport fluid 65 to be injected into axially offset chamber 158 via the circuitous fluid path now to be described. Referring to Figure 21, this circuitous fluid flow path can be seen to comprise a first longitudinally extending fluid passageway 160 which intersects a transversely extending passageway leg 162. Passageway 162 in turn communicates with a longitudinally extending passageway 164 which communicates proximate its upper end with a short transversely extending passageway leg 166. Passageway 166 terminates at the upper portion of chamber 158. With this construction when the flexible side walls of chamber 144 are squeezed, fluid will be injected into chamber 158 via the circuitous passageway thus described following the path indicated by the arrows in Figure 21. Once again chamber 144 is of a sufficient volume to insure that the bibulous portion of the swab 52 will be fully saturated with the transport medium prior to transport of the apparatus to the clinical laboratory for testing. As was the case with the previous described embodiments of the invention, the unique circuitous fluid flow path between chambers 144 and 158 prevents accidental transfer of the transport fluid into the swab containing chamber through vibration, shaking or rough handling of the apparatus.

As indicated in Figures 22, 23 and 24, plastic body 140 is of generally circular cross section at any point. Chamber 158 is also of circular cross section but is axially displaced as indicated in Figure 22. This axially displacement permits the formation of the circuitous passageway with leg 164 of the passageway being structurally supported by the transversely extending web 167 (Figures 21 and 23). Lower chamber 144 is also circular in cross section at any point as is second closure cap 156.

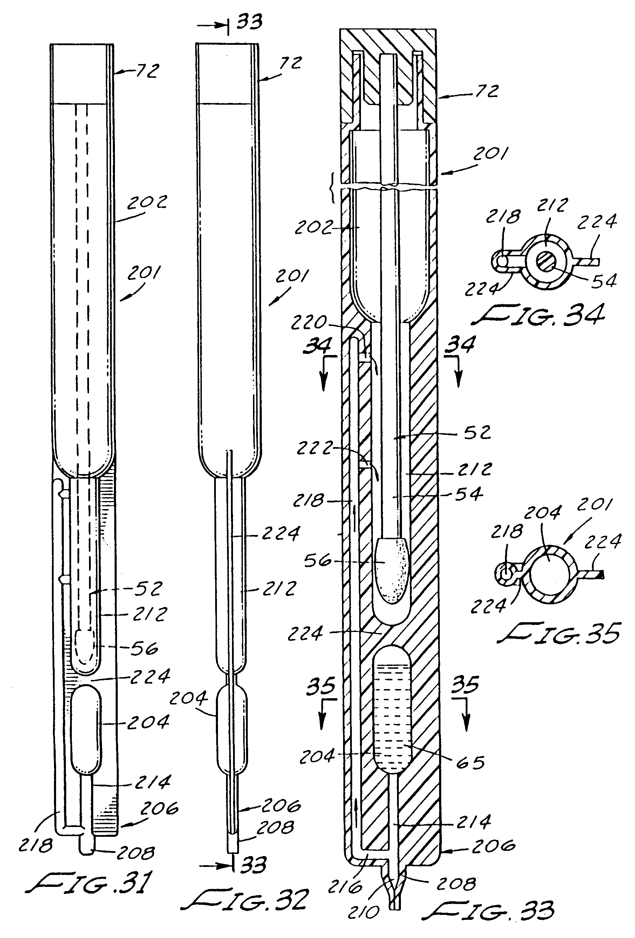

Referring now to Figures 25 through 30, yet another form of the swab form apparatus of the present invention is there shown and generally designated with the numeral 170. The apparatus of this form of the invention is similar in many respects to the embodiments previously described and once again like numbers are used in the drawings to identify like components. The apparatus is used for transporting a swab 52, the stem of which is closely received within a bore formed in a closure cap 72 of a construction identical to that previously described herein.

The apparatus comprises an elongated plastic body 172 having an open end portion 174 which is closed by closure cap 72 and a lower portion 176. Body 172 includes a upper elongated flexible-walled chamber 178 and a lower flexible-walled chamber 180. Disposed intermediate chambers 178 and 180 is a reduced diameter portion provided here in the form of a third flexible-walled chamber 182.

Disposed proximate the second end 176 of the plastic body of a tubular shaped segment 184 having a fluid passageway 186. In the drawings passageway 186 is shown in a sealed condition, segment 184 having been crimped and heat-sealed in a manner previously described. Once again, if desired, a mechanical clamping means can be used to sealably close segment 184.

Chambers 180 and 182 are in fluid communication via a circuitous fluid path comprising a longitudinally extending elongated fluid passageway 188. Passageway 188 is connected proximate its lower end with a transversely extending passageway 190 which communicates with chamber 180 proximate its lower end. Passageway 188 communicates proximate its upper or opposite end with a transversely extending passageway 192 which in turn communicates with chamber 182 proximate the upper end of this chamber.

A highly novel feature of the apparatus of the invention as shown in Figure 27 comprises the provision of a fourth chamber 194. Fourth chamber 194 is in communication with chamber 182 via a fluid passageway comprising a longitudinally extending passageway 196 and a transversely extending passageway 198 which communicates with chamber 182 proximate its lower end. In the present form of the invention, fourth chamber 194 is provided with flexible side walls and is disposed on one side of and in close proximity with chamber 180.

As best seen by referring to Figures 28, 29 and 30, chambers 178, 180, 182 and 194 are all circular in cross section at any point. Passageway 188 is supported by structural reinforcing web 200.

In using the apparatus illustrated in Figures 25 through 30, a fluid such as a transport medium 65, can be drawn into chamber 180 by inserting tubular segment 184 into a vial containing the fluid, squeezing the walls of flexible chamber 180 and then releasing them to cause the fluid 65 to be drawn into chamber 180 in the manner shown in Figure 27. Once the transport medium most appropriate for use in connection with the culture to be taken is drawn into chamber 180, passageway 186 of tubular segment 184 can be heat-sealed in the manner previously described so as to assume the configuration shown in the drawings.

After the specimen has been taken by the physician and the swab reinserted into the apparatus in the manner shown in Figure 27, squeezing the yieldable side walls of chamber 180 will cause the transport fluid to be injected into chamber 182 via passageways 188, 190 and 192. Chamber 180 is of a volume such that when the fluid is transferred from chamber 180 into chamber 182 the fluid will substantially encapsulate the bibulous swab material 56 carried by the swab 52. Once the transport fluid has been injected into chamber 182 a small portion of the culture or specimen residing on the swab material will normally be transferred to the fluid transport medium. By squeezing and then releasing the flexible side walls of chamber 194, a part of the transport fluid now contaminated with the specimen will be drawn into chamber 194 via passageways 196 and 198. By severing the length of tube 197 which interconnects chambers 182 and 194, the contaminated transport fluid contained within chamber 194 and designated in Figure 27 by the numeral 65a can be readily separated from the apparatus. After severing tubular segment 197, chamber 194 can be sealed by heat-sealing or otherwise closing passageway 196 of tubular segment 197. Alternatively, segment 197 can first be heat sealed along a portion of its length and then cut through the sealed portion to separate chamber 194 from the device. This sealing and cutting operation can be accomplished in either one or two steps as desired. The contaminated transport fluid contained within chamber 194 can then be safely transported to the clinical laboratory for testing. This unique feature of the apparatus permits clinical testing of the contaminated transport fluid 65a as well as separate clinical analysis of the contaminated swab 52. Testing of the contaminated fluid 65a may be preliminary to testing of the swab or may allow testing of the specimen by a different method.

Turning finally to Figures 31 through 35, a last embodiment of the invention is thereshown. Many portions of the apparatus of this form of the invention are similar to those described in connection with the apparatus described Figures 13 through 15. However, the relative positioning of the various fluid chambers of the apparatus is somewhat different. The apparatus of this last form of the invention comprises an elongated plastic body portion 201 having a longitudinally extending first or upper chamber 202 and a second longitudinally spaced chamber 204. Chamber 202 is open proximate its upper end and is adapted to sealably receive a closure member or cap 72 carrying a swab assembly 52 of the character previously described. Formed proximate the lower portion 206 of body 200 is a tubular shaped segment 208 having a fluid passageway 210 through which transport fluid can be drawn to fill lower chamber 204. After chamber 204 has been filed with the selected transport fluid medium, tubular segment 208 is heat-sealed in the manner previously described and as indicated in the drawings. Segment 208 can also be sealed using a mechanical clamp if so desired.

Disposed intermediate chambers 202 and 204 is a reduced diameter portion comprising a third flexible-walled elongated chamber 212. As indicated in Figure 33, chambers 202, 204 and 212 are all axially aligned and are in communication with one another via a novel arrangement of fluid passageways of a character presently to be described.

As best seen by referring to Figures 33 through 35, passageway 210 of tubular segment 208 communicates with chamber 204 via an elongated axially extending fluid passageway 214. Intersecting passageway 214 is a transversely extending passageway 216 which communicates at its outer end with an elongated, longitudinally extending passageway 218 which passageway extends on side of chambers 204 and 212. Proximate the upper extremity of passageway 218 is a transversely extending sub-passageway 220 which communicate with chamber 212 proximate its upper end. In the form of the invention shown in Figure 33, a second, longitudinally spaced sub-passageway 222 is also provided. This passageway communicates with chamber 212 proximate the central section thereof. With this arrangement fluid flowing through passageway 218 can enter chamber 212 through sub-passageway 220 as well as through sub-passageway 222. Once again, as indicated in Figures 31 and 33, passageway 218 is structurally supported by a reinforcing web 224.

In using this last form of the apparatus, a fluid such as an appropriate transport medium is either prepackaged with the device or is drawn into chamber 204 in the manner previously described and tubular segments 208 is then clamped or sealed as shown in the drawing. After the specimen has been taken by the physician and the swab reinserted into the apparatus in the manner shown in Figures 31 and 33, squeezing the yieldable side walls of the squeeze bulb or chamber 204 will cause the transport fluid to be injected into chamber 212 via passageways 216, 218, 220 and 222. Chamber 204 is of such a volume that when the fluid is transferred into chamber 212 the fluid will substantially encapsulate the bibulous swab material 56 of swab 52 which is disposed proximate the lower extremity of chamber 212. With the fluid transport medium having saturated the bibulous swab material, the entire apparatus can be safely transported to the clinical laboratory for testing.

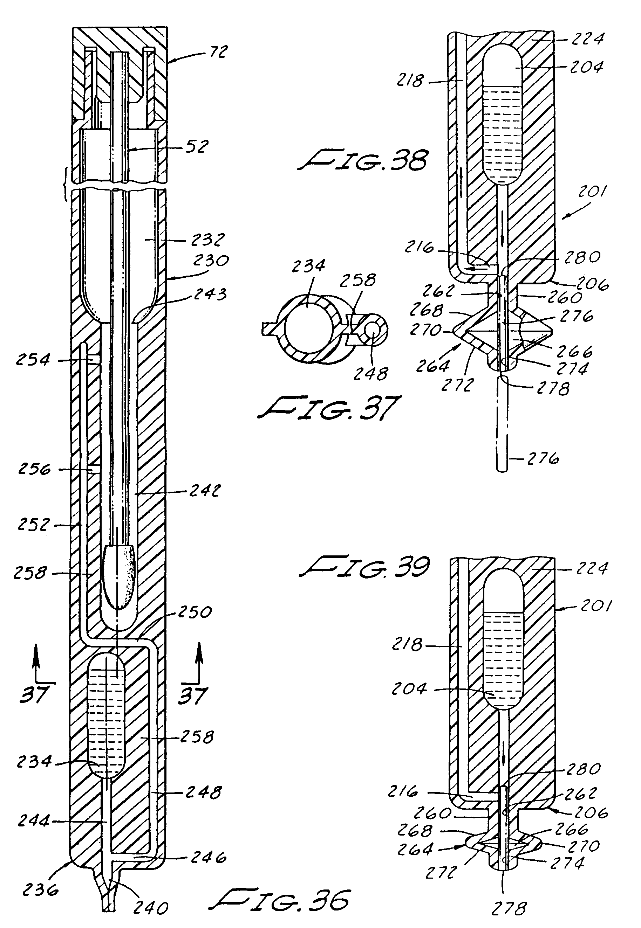

As indicated in Figures 34 and 35, once again plastic body 200 of the apparatus is substantially circular in cross section at any point as are chambers 204 and 212.

A particular advantage of this last to be described embodiment of the invention resides in the placement of chambers 212 and 204 and their interconnection through the novel circuitous flow path defined by passageways 214, 216, 218, 220 and 222. The location of chambers 204 and 212, coupled with the configuration of the circuitous flow path, positively precludes accidental transfer of the fluid medium from chamber 204 into 212 as a result of handling, shock or vibration of the apparatus of the invention. For example, even through violent shaking of the apparatus of this last form of the invention, the fluid in chamber 204 cannot reach chamber 212. Rather, the transfer of fluid can be accomplished only by positive manipulation of the flexible outer walls of chambers 204 and 212.

Turning to Figures 36 and 37, a further embodiment of the invention is thereshown. Many portions of the apparatus of this form of the invention are similar to those described in connection with the apparatus described in Figures 31 through 39. However, the relative positioning of the various fluid chambers of the apparatus is somewhat different. The apparatus of this further form of the invention comprises an elongated plastic body portion 230 having a longitudinal axis, a longitudinally extending first or upper chamber 232 and a second longitudinally spaced chamber 234. Chamber 232 is open proximate its upper end and is adapted to sealably receive a closure member or cap 72 carrying a swab assembly 52 of the character previously described. It is to be observed that chamber 234 is axially offset from the longitudinal axis of chamber 232. Formed proximate the lower portion 236 of body 230 is a tubular shaped segment 238 having a fluid passageway 240 through which transport fluid can be drawn to fill lower chamber 234. After chamber 234 has been filled with the selected transport fluid medium, tubular segment 238 is heat-sealed in the manner previously described. Segment 238 can also be sealed using a mechanical clamp if so desired.

Disposed intermediate chambers 232 and 234 is a reduced diameter portion comprising a third flexible-walled elongated chamber 242. As indicated in Figure 36, chambers 232 and 242 are axially aligned and are in communication with one another via the open juncture 243 of the chambers.

As best seen by referring to Figure 36, passageway 240 of tubular segment 238 communicates with chamber 234 via an elongated axially extending first fluid passageway 244. Intersecting passageway 244 is a first transversely extending passageway 246 which communicates at its outer end with an elongated, longitudinally extending second passageway 248 which passageway extends on the right side of chamber 234 as viewed in Figure 36. Passageway 248 connects with a second transverse passageway 250 which, in turn, connects with a third longitudinally extending passageway 252, which passageway extends on the left side of chamber 242 as viewed in Figure 36. Proximate the upper extremity of passageway 252 is a third transversely extending passageway 254 which communicates with chamber 242 proximate its upper end. In the form of the invention shown in Figure 36, a fourth transversely extending passageway 256 is also provided. This passageway communicates with chamber 242 proximate the central section thereof. With this arrangement fluid flowing through passageway 252 can enter chamber 242 through passageway 254 as well as through passageway 256. As indicated in Figures 36 and 37, passageway 248 and 252 are structurally supported by a reinforcing web 258.

The form of the apparatus described in the preceding paragraphs is used in substantially the same manner as the embodiment of the invention shown in Figures 31 through 35. Referring now to Figures 38 and 39, yet another embodiment of the apparatus of the invention is there illustrated. This embodiment of the apparatus is similar in most respects to the apparatus shown in Figures 33 through 35 and only the lower most portions of the apparatus are illustrated in Figures 38 and 39. However, it is to be understood that this form of the invention includes an elongated plastic body portion 201 having a longitudinal axis, a longitudinally extending first or upper chamber 202 and a second longitudinally spaced chamber 204. Chamber 202 is open proximate its upper end and is adapted to sealably receive a closure member or cap 72 carrying a swab assembly 52 of the character previously described (see Figure 33).

Form proximate the lower portion 206 of body 201 is a tubular shaped segment having a fluid passageway 262 therethrough. Integrally formed with segments 260 is the unique closure means of the present invention for blocking flow of fluid from chamber 204 through passageway 216. The details of this closure means will presently be described.

Disposed intermediate chambers 202 and 204 is a reduced diameter portion comprising a third flexible walled elongated chamber 212 (Figure 33). Chambers 202 and 212 are in communication with one another via the open juncture of the chambers.

As best seen by referring to Figures 33 and 38, passageway 262 of tubular segment 260 communicates with chamber 212 via a transversely extending passageway 216 and a laterally extending passageway 218 (Figure 33).

The closure means of this last embodiment of the apparatus of the invention functions to block the view of fluid from chamber 204 through passageways 262, 216 and 218 toward chamber 212. In the present form of the invention, this closure means comprises a collapsible bellows 264 having a chamber 266 in communication with passageway 262 of tubular segment 260. Bellows 264 includes an upper portion having outwardly sloping first side walls 268 terminating in a generally annular shaped portion 270. Bellows 264 also includes a lower portion having outwardly sloping second side walls 272 terminating in a generally annular shaped portion interconnecting with said first generally annular shaped portion defined by upper walls 268.

Second side walls 272 have an aperture 274 formed therein.

As illustrated in Figures 38 and 39, bellows 264 is movable from a first expanded position as shown in Figures 38 to a second collapsed position as shown in Figure 9 wherein second walls 272 are moved into close proximity with first walls 268.

Also forming a part of the closure means of this embodiment of the invention is an elongated closure plug 276 having first and second ends 278 and 280 respectively.

Plug 276 is removably receivable within aperture 274 of the second side walls of the bellows and is also slidably receivable within passageway 262 of tubular segment 260. When plug 276 is removed from the bellows structure as indicated by the phantom lines in Figure 38, chamber 204 can be filled with a selected transport fluid medium. This done, plug 276 is inserted into the bellows assembly in the manner shown in Figure 38 and bonded in place either by an adhesive or by heat sealing walls 272 in the area of aperture 274. With plug 276 affixed in place within aperture 274, movement of the bellows from the expanded position shown in Figure 238 to the compressed position shown in Figure 39 will cause second end 280 of plug 276 to move upwardly within passageway 262 to a position wherein transverse passageway 216 is blocked so that fluid within chamber 204 cannot flow toward chamber 212.

In using the swab transport device of this latter form of the invention, once the fluid transport medium is added to chamber 204, the bellows can be compressed in the manner shown in Figure 39 moving plug 276 into a position wherein fluid flow through the circuitous passageway leading to 212 is positively blocked. With the plug in this position accidental transfer of fluid from chamber 204 to chamber 212 is rendered virtually impossible. When it is desired to positively transfer fluid from chamber 204 through the circuitous pathway into chamber 212 the bellows assembly can once again be expanded to the position shown in Figure 38, and the chambers appropriately manipulated to cause positive flow of fluid from chamber 204 into chamber 212 thereby saturating swab 256.

In the preferred form of the invention, the body portion 201, the tubular body segment 260 and the bellows construction are all integrally formed, e.g. of an unsaturated hydrocarbon material selected from the polyolefin group.

## Claims

1. A swab transport apparatus for containing and transporting a swab (52) of the character having an elongated stem (54) and a bibulous swab material (56) carried proximate one end of the stem, said apparatus comprising an elongated plastic body (58; 86; 110, 140; 172; 201; 230) having a longitudinally extending axis, resiliently deformable side walls and first and second ends (60, 61; 88, 90; 114, 116; 146, 150; 174, 176; 206; 236), said body being open at said first end (60; 88; 114; 146; 174) and including:
(a) a first elongated chamber (62; 92; 112; 142; 178; 202; 232) located proximate said first open end for receiving at least a portion of the swab;
(b) a second chamber (64; 94; 120; 144; 180; 204; 234) having resiliently, deformable side walls for containing fluid therewithin, said second chamber being longitudinally displaced from said first chamber; and
(c) a tubular segment (68; 98; 118; 148; 184; 208; 238; 260) disposed proximate said second end of said body, said segment having a fluid passageway (70; 100; 152; 186; 210; 240; 262) extending therethrough for interconnecting said second chamber with atmosphere, characterised in that a reduced diameter portion (66; 96; 122; 158; 182; 212; 242) is disposed intermediate said first and second chambers, said reduced diameter portion being in communication with both said first and second chambers, wherein said
reduced diameter portion includes a third chamber (96; 122; 158; 182; 212; 242) having resiliently deformable side walls disposed intermediate said first and second chambers, said third chamber being in communication with said first chamber via an axially extending fluid passageway and said third chamber being in communication with said second chamber via a circuitous fluid flow path (102, 104, 106; 124, 126, 128, 130; 160, 162, 164, 166; 188, 190, 192; 214, 216, 218, 220, 222; 244, 246, 248, 250, 252, 254, 256).

2. Apparatus as in claim 1, in which said first and third chambers (232, 242) are axially aligned, and said second chamber (234) is axially offset from said first chamber.

3. Apparatus as in claim 1, in which said first and second chambers (142, 144) are axially aligned and in which said third chamber (158) is axially offset with respect to said first and second chamber.

4. Apparatus as in any one of claims 1-3, further comprising a removable first cover means (72) for sealably closing said open first end (146) of said tube, said first cover means including an axially extending bore for closely receiving a portion of the elongated stem (54) of the swab (52); and
a removable second cover means (156) carried by said tubular segment for surrounding said second chamber (144) to prevent inadvertent deformation of the side walls thereof.

5. Apparatus as in claim 4, in which said second cover means (156) comprises a cup shaped member having curved outer walls and an aperture formed in said walls for closely receiving said tubular segment.

6. Apparatus as in claim 1, 3, 4 or 5, in which said second chamber (234) is in communication with said first fluid passageway (240) of said tubular segment (238) and in which said third chamber (242) is in communication with said second chamber (234) via a circuitous passageway having a first transversely extending leg (246), a second longitudinally extending leg (248), a second transversely extending leg (250), a third longitudinally extending leg (252) and a third transversely extending leg (254), said passageway defining a circuitous fluid flow path.

7. Apparatus as in claim 6 in which said circuitous passageway also includes a fourth transversely extending leg (256) in communication with said third longitudinally extending leg (252).

8. Apparatus as in claim 6 or 7, in which said second transversely extending leg (250) is disposed intermediate said second and third chambers (234, 242).

9. Apparatus as in claim 1, 3, 4 or 5, in which a portion (102; 124, 128; 160, 164; 188; 214, 218; 244, 248, 252) of said circuitous fluid flow path extends parallel to the longitudinal axis of said third chamber and a portion (104, 106; 126, 130; 162, 166; 190, 192; 216, 220, 222; 246, 250, 254, 256) of said circuitous fluid flow path extends perpendicular to the axis of said third chamber.

10. Apparatus as in claim 6, 7, 8 or 9 further comprising closure means carried by said body for closing said circuitous passageway, said closure means comprising:
(i) a collapsible bellows (264) having a chamber (266) in communication with said passageway (262) of said tubular segment (260), said bellows including an upper portion having outwardly sloping first side walls (268) terminating in a generally annular shaped portion (270), and a lower portion having outwardly sloping second side walls (272) terminating in a generally annular shaped port interconnected with said first generally annularly shaped portion (270) of said upper portion, said second side walls (272) having an aperture (274) therethrough, said bellows being movable from a first expanded position wherein said outwardly sloping first walls of said upper portion are spaced apart from said outwardly sloping second walls of said lower portion to a second collapsed position wherein said second walls are proximate said first walls; and
(ii) an elongated plug (276) removably receivable within said aperture (274) of said second side walls (272) of said bellows, one end of said plug being slidably receivable within said passageway (262) of said segment (260) and being movable from a first position spaced apart from said circuitous passageway to a second position blocking said circuitous passageway.

11. Apparatus as in claim 10 in which said tubular body segment (260) and said collapsible bellows (264) are integrally formed of an unsaturated hydrocarbon material selected from the polyolefin group.

12. Apparatus as in claim 1, further including a fourth chamber (194) having resiliently deformable side walls, said fourth chamber being in communication with said third chamber (192).

13. Apparatus as in claim 12, in which said fourth chamber (194) is disposed proximate to and includes a longitudinal central axis extending generally parallel with respect to a central axis of said second chamber (180).

14. Apparatus as in claim 12 or 13, in which said second and fourth chambers (180, 194) are interconnected by an axially extending tubular member.

## Patentansprüche

1. Wattestäbchen-Transportvorrichtung zum Aufnehmen und Transportieren eines Wattestäbchens (52) der Art, die ein längliches Stäbchen (54) und ein saugfähiges Bauschmaterial (56) hat, das nahe an einem Ende des Stäbchens getragen ist, wobei die Vorrichtung einen länglichen Kunststoffkörper (58; 86; 110, 140; 172; 201; 230) umfaßt, der eine längs verlaufende Achse, elastisch verformbare Seitenwände und erste und zweite Enden (60, 61; 88, 90; 114, 116; 146, 150; 174, 176; 206; 236) hat, wobei der Körper an einem ersten Ende (60; 88; 114; 146; 174) offen ist und umfaßt:
(a) eine erste längliche Kammer (62; 92; 112; 142; 178; 202; 232), die in der Nähe des ersten offenen Endes zur Aufnahme zumindest eines Bereichs des Wattestäbchens angeordnet ist,
(b) eine zweite Kammer (64; 94; 120; 144; 180; 204; 234), die elastische verformbare Seitenwände zum darin Aufnehmen eines Fluids hat, wobei die zweite Kammer in Längsrichtung von der ersten Kammer versetzt ist, und
(c) ein rohrförmiges Segment (68; 98; 118; 148; 184; 208; 238; 260), das in der Nähe des zweiten Endes des Körpers angeordnet ist, wobei das Segment einen Fluiddurchlaß (70; 100; 152; 186; 210; 240; 262) hat, der sich durch das Segment hindurch zur Verbindung der zweiten Kammer mit der Atmosphäre verstrickt,
dadurch gekennzeichnet, daß
ein durchmesserreduzierter Bereich (66; 96; 122; 158; 182; 212; 242) zwischen den ersten und zweiten Kammern angeordnet ist, wobei der durchmesserreduzierte Bereich in Verbindung mit sowohl der ersten wie der zweiten Kammer steht, wobei der durchmesserreduzierte Bereich eine dritte Kammer (96; 122; 158; 182; 212; 242) umfaßt, die elastisch verformbare Seitenwände hat, die zwischen den ersten und zweiten Kammern angeordnet sind, wobei die dritte Kammer in Verbindung mit der ersten Kammer über einen axial verlaufenden Fluiddurchlaß steht, und wobei die dritte Kammer in Verbindung mit der zweiten Kammer über einen Umlauffluidströmungspfad (102, 104, 106; 124, 126, 128, 130; 160, 162, 164, 166; 188, 190, 192; 214, 216, 218, 220, 222; 244, 246, 248, 250, 252, 254, 256) steht.

2. Vorrichtung nach Anspruch 1, bei der die ersten und dritten Kammern (232, 242) axial ausgerichtet sind, und bei der die zweite Kammer (234) von der ersten Kammer axial versetzt ist.

3. Vorrichtung nach Anspruch 1, bei der die ersten und zweiten Kammern (142, 194) axial ausgerichtet sind, und bei der die dritte Kammer (158) axial in bezug auf die ersten und zweiten Kammern versetzt ist.

4. Vorrichtung nach einem der Ansprüche 1 bis 3, zusätzlich umfassend eine entfernbare erste Abdeckeinrichtung (72) zum dichtenden Verschließen des offenen ersten Endes (156) des Rohrs, wobei die erste Abdeckeinrichtung eine axial verlaufende Bohrung zum festsitzenden Aufnehmen eines Bereichs des länglichen Stäbchens (54) des Wattestäbchens (52) einschließt, und eine zweite Abdeckeinrichtung (156), die durch das rohrförmige Segment zum Umschließen der zweiten Kammer (144) getragen ist, um eine unbeabsichtigte Verformung ihrer Seitenwände zu verhindern.

5. Vorrichtung nach Anspruch 4, bei der die zweite Abdeckeinrichtung (156) ein kappenförmiges Element umfaßt, das gekrümmte Außenwände und eine in den Wänden zur festsetzenden Aufnahme des rohrförmigen Segments ausgebildete Öffnung hat.

6. Vorrichtung nach Anspruch 1, 3, 4 oder 5, bei der die zweite Kammer (234) in Verbindung mit dem ersten Fluiddurchlaß (240) des rohrförmigen Segments (238) steht, und bei der die dritte Kammer (242) in Verbindung mit der zweiten Kammer (234) über einen Umlaufdurchlaß steht, der einen ersten in Querrichtung verlaufenden Schenkel (246) hat, einen zweiten in Längsrichtung verlaufenden Schenkel (248), einen zweiten in Querrichtung verlaufenden Schenkel (250), einen dritten in Längsrichtung verlaufenden Schenkel (252) und einen dritten in Querrichtung verlaufenden Schenkel (254), wobei die Durchlässe einen Umlauffluidströmungspfad festlegen.

7. Vorrichtung nach Anspruch 6, bei der der Umlaufdurchlaß einen vierten in Querrichtung verlaufenden Schenkel (256) einschließt, der in Verbindung mit dem dritten in Längsrichtung verlaufenden Schenkel (252) steht.

8. Vorrichtung nach Anspruch 6 oder 7, bei der der zweite in Querrichtung verlaufende Schenkel (250) zwischen den ersten und zweiten Kammern (234, 242) angeordnet ist.

9. Vorrichtung nach Anspruch 1, 3, 4 oder 5, bei der ein Bereich (102; 124, 128; 160, 164; 188; 214, 218; 244, 248, 252) des Umlauffluidströmungspfads parallel zur Längsachse der dritten Kammer und ein Bereich (104, 106; 126, 130; 162, 166; 190, 192; 216, 220, 222; 246, 250, 254, 256) des Umlauffluidströmungspfads quer zur Achse der dritten Kammer verläuft.

10. Vorrichtung nach Anspruch 6, 7, 8 oder 9, zusätzlich umfassend eine Verschlußeinrichtung, die durch den Körper zum Verschließen des Umlaufdurchlasses getragen ist, wobei die Verschlußeinrichtung umfaßt:
(i) einen zusammenfaltbaren Balg (264) mit einer Kammer (266), die in Verbindung mit dem Durchlaß (262) des rohrförmigen Segments (260) steht, wobei der Balg einen oberen Bereich einschließt, der nach außen abgeschrägte erste Seitenwände (268) hat, die in einem allgemein ringförmigen Bereich (270) enden, und einen unteren Bereich, der nach außen abgeschrägte zweite Seitenwände (272) hat, die in einer allgemein ringförmigen Öffnung enden, die mit dem ersten allgemein ringförmigen Bereich (270) des oberen Bereichs in Verbindung steht, wobei die zweiten Seitenwände (272) eine Öffnung (274) durch sie hindurch haben, wobei der Balg aus einer ersten gestreckten Position, in der die nach außen abgeschrägten ersten Wände des oberen Bereichs von den nach außen abgeschrägten zweiten Wänden des unteren Bereichs beabstandet sind, in eine zweite zusammengefaltete Position beweglich ist, in der die zweiten Wände nahe zu den ersten Wänden liegen; und
(ii) einen länglichen Stopfen (276), der in der Öffnung (274) der zweiten Seitenwände (272) des Balgs entfernbar aufgenommen ist, wobei ein Ende des Stopfens in dem Durchlaß (262) des Segments (260) gleitend aufnehmbar und aus der ersten Position, die von dem Umlaufdurchlaß beabstandet ist, in eine zweite Position beweglich ist, die den Umlaufdurchlaß blockiert.

11. Vorrichtung nach Anspruch 10, bei der das ringförmige Körpersegment (260) und der zusammenfaltbare Balg (264) aus einem Material ungesättigten Kohlenwasserstoffs integral ausgebildet sind, der aus der Polyolefingruppe ausgewählt ist.

12. Vorrichtung nach Anspruch 1, zusätzlich umfassend eine vierte Kammer (194) mit elastisch verformbaren Seitenwänden, wobei die vierte Kammer in Verbindung mit der dritten Kammer (192) steht.

13. Vorrichtung nach Anspruch 12, bei der die vierte Kammer (194) in der Nähe zu einer Mittenachse der zweiten Kammer (180) angeordnet ist und eine Mittenlängsachse einschließt, die allgemein parallel zu der Mittenachse der zweiten Kammer (180) verläuft.

14. Vorrichtung nach Anspruch 12 oder 13, bei der die zweiten und vierten Kammern (180, 194) miteinander durch ein axial verlaufendes rohrförmiges Element verbunden sind.

## Revendications

1. Un dispositif pour le transport de tampon, destiné à contenir et transporter un tampon (52) du type ayant une tige allongée et un matériau hydrophile (56) de tampon situé à proximité d'une extrémité de la tige, ledit dispositif comprenant un corps plastique allongé (58 ; 86; 110 ; 140; 172 ; 201 ; 230) ayant un axe longitudinal, des parois latérales déformables de manière élastique et les première et seconde extrémités (60, 61 ; 88, 90 ; 114, 116; 146, 150 ; 174, 176 ; 206 ; 236), ledit corps étant ouvert à ladite première extrémité (60 ; 88 ; 114 ; 146 ; 174) et comprenant :
(a) une première chambre allongée (62 ; 92 ; 112; 142 ; 178 ; 202 ; 232) située à proximité de ladite première extrémité ouverte, destinée à recevoir au moins une partie du tampon ;
(b) une seconde chambre (64 ; 94 ; 120 ; 144 ; 180 ; 204 ; 234) ayant des parois déformables de manière élastique pour contenir un fluide, ladite seconde chambre étant écartée longitudinalement par rapport à ladite première chambre ; et
(c) un segment tubulaire (68 ; 98 ; 118 ; 148; 184 ; 208 ; 238 ; 260) disposé à proximité de ladite seconde extrémité dudit corps, ledit segment étant traversé par un passage pour le fluide (70 ; 100 ; 152 ; 186 ; 210; 240; 262), destiné à relier ladite seconde chambre à l'atmosphère,
caractérisé en ce qu'une partie de diamètre réduit (66; 96 ; 122 ; 158 ; 182 ; 212 ; 242) est placée entre lesdites première et deuxième chambres, ladite partie d'un diamètre réduit étant en communication avec ces deux première et seconde chambres,
où ladite partie d'un diamètre réduit comprenant une troisième chambre (96 ; 122; 158 ; 182 ; 212 ; 242) ayant des parois déformables de manière élastique, placée entre lesdites première et seconde chambres, ladite troisième chambre étant en communication avec ladite première chambre par un passage pour le fluide, disposé de manière axiale, et ladite troisième chambre étant en communication avec ladite seconde chambre par une voie d'écoulement du fluide en forme de circuit non direct (102, 104, 106 ; 124, 126, 128, 130 ; 160, 162, 164, 166 ; 188, 190, 192 ; 214, 216, 218, 220, 222 ; 244, 246, 248, 250, 252, 254, 256).

2. Un dispositif selon la revendication 1 dans lequel lesdites première et troisième chambres (232, 242) sont axialement alignées, et ladite seconde chambre (234) est décalée axialement par rapport à ladite première chambre.

3. Un dispositif selon la revendication 1 dans lequel lesdites première et seconde chambres (142, 144) sont axialement alignées, et ladite troisième chambre (158) est décalée axialement par rapport auxdites première et seconde chambres.

4. Un dispositif selon l'une quelconque des revendications 1 à 3, comprenant en outre un premier couvercle amovible (72) destiné à fermer de manière étanche ladite première extrémité ouverte (146) dudit tube, ledit premier couvercle comprenant un alésage s'étendant axialement pour recevoir en contact étroit une partie de la tige allongée (54) du tampon (52) ; et
un second couvercle amovible (156) porté par ledit segment tubulaire destiné à entourer ladite seconde chambre (144) afin d'empêcher la déformation par inadvertance des parois latérales de celle-ci.

5. Un dispositif selon la revendication 4 dans lequel ledit second couvercle (156) comprend un élément en forme de cuvette ayant des parois extérieures cintrées et une ouverture formée dans lesdites parois pour recevoir étroitement ledit segment tubulaire.

6. Un dispositif selon la revendication 1, 3, 4 ou 5 dans lequel ladite seconde chambre (234) est en communication avec ledit premier passage pour le fluide (240) dudit segment tubulaire (238) et dans lequel ladite troisième chambre (242) est en communication avec ladite seconde chambre (234) par un passage en forme de circuit non direct ayant une première branche transversale (246), une seconde branche longitudinale (248), une seconde branche transversale (250), une troisième branche transversale (252) et une troisième branche longitudinale (254), ledit passage définissant une voie d'écoulement du fluide en forme de circuit non direct.

7. Un dispositif selon la revendication 6 dans lequel ledit passage en forme de circuit comporte aussi une quatrième branche transversale (256) en communication avec ladite troisième branche longitudinale (252).

8. Un dispositif selon la revendication 6 ou 7 dans lequel ladite seconde branche transversale (250) est placée entre lesdites seconde et troisième chambres (234, 242).

9. Un dispositif selon la revendication 1, 3, 4 ou 5 dans lequel une partie (102 ; 124, 128 ; 160, 164 ; 188 ; 214, 218 ; 244, 248, 252) de ladite voie d'écoulement du fluide en forme de circuit s'étend parallèlement à l'axe longitudinal de ladite troisième chambre et une partie (104, 106 ; 126, 130 ; 162, 166 ; 190, 192 ; 216, 220, 222; 246, 250, 254, 256) de ladite voie d'écoulement en forme de circuit s'étend perpendiculairement par rapport à l'axe de ladite troisième chambre.

10. Un disposition selon la revendication 6, 7, 8 ou 9 comprenant en outre un moyen de fermeture porté par ledit corps pour fermer ledit passage serpentant, ledit moyen de fermeture comprenant :
(1) un soufflet repliable (264) ayant une chambre (266) en communication avec ledit passage (262) dudit segment tubulaire (260), ledit soufflet comportant une partie supérieure ayant des premières parois latérales (268) inclinées vers l'extérieur se terminant en une partie de forme générale annulaire (270), et une partie inférieure ayant des secondes parois latérales (272) inclinées vers l'extérieur se terminant en une partie de forme générale annulaire reliée à ladite première partie de forme générale annulaire (270) de ladite partie supérieure, lesdites secondes parois latérales (272) étant traversées par une ouverture (274), ledit soufflet étant mobile à partir d'une première position d'extension, dans laquelle lesdites premières parois inclinées vers l'extérieur de ladite paroi supérieure sont espacées desdites secondes parois inclinées vers l'extérieur de ladite partie inférieure jusqu'à une seconde position dans laquelle lesdites secondes parois sont proches desdites premières parois ; et
(ii) un bouchon allongé (276) entrant de manière amovible dans ladite ouverture (274) desdites secondes parois latérales (272) dudit soufflet, une extrémité dudit bouchon entrant par coulissement dans ledit passage (262) dudit segment (260) et étant mobile depuis une première position écartée dudit passage en forme de circuit en une seconde position bloquant ledit passage en forme de circuit.

11. Un dispositif selon la revendication 10 dans lequel ledit segment du corps tubulaire (260) et ledit soufflet repliable (264) sont intégralement formés d'un matériau hydrocarboné insaturé choisi parmi le groupe des polyoléfines.

12. Un dispositif selon la revendication 1, comprenant en outre une quatrième chambre (194) ayant des parois latérales déformables de manière élastique, ladite quatrième chambre étant en communication avec ladite troisième chambre (192).

13. Un dispositif selon la revendication 12, dans lequel ladite quatrième chambre (194) est placée à proximité de la seconde chambre (180), et comprend un axe central longitudinal s'étendant dans l'ensemble en parallèle par rapport à un axe central de ladite seconde chambre (180).

14. Un dispositif selon la revendication 12 ou 13, dans lequel lesdites seconde et quatrième chambres (180, 194) sont reliées par un élément tubulaire s'étendant axialement.
